Europäisches Patentamt

European Patent Office

Office européen des brevets

(19)

(11) Veröffentlichungsnummer: **0 407 788 A2**

(12) **EUROPÄISCHE PATENTANMELDUNG**

(21) Anmeldenummer: **90111908.1**

(51) Int. Cl.5: **A61K 31/05**

(22) Anmeldetag: **22.06.90**

| | |
|---|---|
| Die Bezeichnung der Erfindung wurde geändert (Richtlinien für die Prüfung im EPA, A-III, 7.3).<br><br>(30) Priorität: **06.07.89 CH 2520/89**<br><br>(43) Veröffentlichungstag der Anmeldung:<br>**16.01.91 Patentblatt 91/03**<br><br>(84) Benannte Vertragsstaaten:<br>**AT BE CH DE DK ES FR GB GR IT LI LU NL SE** | (71) Anmelder: **F. HOFFMANN-LA ROCHE AG**<br>**Postfach 3255**<br>**CH-4002 Basel(CH)**<br><br>(72) Erfinder: **Bollag, Werner, Dr.**<br>**Mythenstrasse 10**<br>**CH-4054 Basel(CH)**<br><br>(74) Vertreter: **Lederer, Franz, Dr. et al**<br>**Patentanwalt Dr. Franz Lederer**<br>**Lucile-Grahn-Strasse 22**<br>**D-8000 München 80(DE)** |

(54) **Verwendung von Retinoiden zur Behandlung und Prophylaxe von Immunkrankheiten.**

(57) p-[2-(5,6,7,8-Tetrahydro-5,5,8,8-tetramethyl-2-naphthyl)propenyl]phenol ist bei immunologischen Indikationen wirksam.

EP 0 407 788 A2

# VERWENDUNG VON RETINOIDEN

Die vorliegende Erfindung betrifft eine neue medizinische Verwendung des p-[2-(5,6,7,8-Tetrahydro-5,5,8,8,-tetramethyl -2-(naphthyl)propenyl]phenols.

Die Verbindung kann als E- oder Z-Isomer oder in Form eines Gemisches beider Isomeren vorliegen. Im Rahmen der vorliegenden Erfindung ist das E-Isomer bevorzugt.

Das p-[2-(5,6,7,8-Tetrahydro-5,5,8,8-tetramethyl-2-naphthyl)propenyl]phenol, seine Herstellung und Verwendung als Heilmittel bei Präkanzerosen und Karzinomen der Haut, der Schleimhäute und inneren Organe sowie bei der topischen und systemischen Therapie von Akne, Psoriasis und anderen mit pathologisch veränderten Verhornung der Haut und von rheumatischen Erkrankungen, insbesondere solcher entzündlicher oder degenerativer Art ist aus der europäischen Patentschrift Nr. 232 779 bekannt.

Ueberraschend wurde gefunden, dass die obengenannte Verbindung zur Behandlung oder Prophylaxe von Krankheiten geeignet ist, die durch physiologische oder pathologische Ver änderungen der Aktivitäten des Immunsystems hervorgerufen werden. Insbesondere sind dies Organtransplantat-Abstossung, graft-versus-host disease, durch T-Zellen vermittelte immunpathologische Erkrankungen, Autoimmunkrankheiten,durch humorale Antikörper hervorgerufene immunpathologische Erkrankungen, allergische Erkrankungen, und durch Immunkomplexe hervorgerufene Erkrankungen. Spezifische Beispiele derartiger Erkrankungen sind Hauterkrankungen wie Lichen planus, Alopecia areata und totalis, Dermatomyositis, Skleroderma, discoider Lupus erythematosus, Vitiligo, Kontaktdermatitis, Ekzeme, atopische Dermatitis und Pemphigus vulgaris; und Erkrankungen anderer Organe, wie Rhinitis allergica, Asthma bronchiale, Hashimoto's Erkrankung, Basedow'sche Krankheit, Myasthenia gravis, Behcet's Erkrankung, rezidivierende aphthöse Stomatitis, Uveitis, primäre biliäre Hepatitis, aktive chronische Hepatitis, Morbus Crohn, Insulin-abhängiger Diabetes mellitus vom Typ I, multiple Sklerose, systemischer Lupus erythematosus, Reitersche Krankheit, autoimmune hämolytische Anämie, idiopathische thrombocytopenische Purpura und idiopathische Leukopenie. In in-vitro Versuchen konnte nachgewiesen werden, dass die Proliferation von immunkompetenten Zellen und die Sekretion von Cytokinen wie Interleukin-2, $\gamma$-Interferon, TNF-$\alpha$ sowie von Immunglobulinen durch das p-[(E)-2-(5,6,7,8-Tetrahydro -5,5,8,8-tetramethyl-2-naphthyl)propenyl]phenol (nachfolgend als "Verbindung A" bezeichnet) moduliert bzw. unterdrückt wird. Diese Versuche werden nachstehend weiter erläutert.

I. Wirkung von unterschiedlichen Konzentrationen der Verbindung A auf Mitogen-stimulierte periphere Blutlymphozyten (PBLC)

Frische, antikoagulierte (Heparin oder EDTA) Blutproben von nicht ausgewählten Probanden wurden zentrifugiert (15 Minuten/500 x g/22° C), das Plasma abpipettiert und durch Kulturmedium (RPMI-1640 mit 100 ml/l hitzeinaktiviertem (30 Minuten/56° C) fötalem Kälberserum, 4 mMol/l 1-Glutamin, 15 mMol/l Hepes, 100 IE/ml Penicillin, 100$\mu$g/ml Streptomycin - alle Komponenten von GIBCO - und 100 mg/l Heparin) ergänzt.

Die Blutzellen wurden mit Kulturmedium im Verhältnis 1:10 gemischt und zusammen mit unterschiedlichen Konzentrationen der Verbindung A bei Anwesenheit von Mitogenen (0-10 $\mu$g/ml Phythämagglutinin resp. 0-20 $\mu$g/ml Concanavalin A) in Teströhrchen (Falcon N° 2058) während 4-7 Tagen bei 37° C in einem 5% $CO_2$-haltigen Luftgemisch und 100% relativer Feuchtigkeit inkubiert. Anschliessend wurde in den Zellüberständen der Gehalt an produziertem Human $\gamma$-Interferon enzymimmunologisch bestimmt [Gallati et al.,J. Biol. Regulators and Homeostatic Agents Vol. 1, 109-118 (1987)].

Die Resultate sind in der Tabelle 1 dargestellt.

Tabelle 1

gamma-IFN [ng/ml]

| M | gamma-IFN [ng/ml] |
|---|---|
| Kontrolle | 100 |
| $1{,}6 \cdot 10^{-6}$ | 68.5 |
| $6{,}25 \cdot 10^{-6}$ | 57 |
| $1{,}25 \cdot 10^{-5}$ | 50 |
| $2{,}5 \cdot 10^{-5}$ | 9.5 |
| $5 \cdot 10^{-5}$ | 0 |
| $1 \cdot 10^{-4}$ | 0 |

II. Hemmung der Proliferation mononukleärer Zellen aus peripherem Blut und der Sekretion der Cytokine Interleukin-2, $\gamma$-Interferon und TNF-$\alpha$

Mononukleäre Blutzellen von peripherem Blut (PBMC) wurden aus heparinisiertem Vollblut mittels Ficoll-Hypaque-Zentrifugierung isoliert. Die Zellkonzentration wurde auf $10^6$-Zellen pro ml in RPMI-haltigem 10%-igem Humanserum eingestellt und in 100 µl-Anteilen in Gegenwart von PHA (2 µg/ml) auf Testplatten verteilt. Reihenverdünnungen (1:3) der Verbindung A wurden in 3 Versuchsreihen in 100 µl-Volumina zugesetzt. Nach 2 Tagen wurden die Kulturen mit 1 µci $^3$H-Thymidin versetzt und der Einbau des markierten Nucleotids nach weiteren 16 Stunden gemessen.

Zur Bestimmung der Cytokinproduktion wurden die Zellen (PBMC) wie oben mit PHA kultiviert. Die Verbindung A wurde in eine Konzentration von $10^{-5}$M zugesetzt. Die Kulturüberstände wurden nach 48 Stunden zur Bestimmung von IL-2, $\gamma$-IFN und TNF-$\alpha$ gesammelt. Die IL-2-Aktivität wurde mit der IL-2-abhängigen T-Zellinie CTLL als Antwortzelle bestimmt (Gillis et al., J. Immunol. 120:2027, 1978).

Wie aus der Tabelle 2 ersichtlich, wurde die Proliferation der Zellen stark unterdrückt; die Sekretion der Cytokine wurde stark bis fast vollständig unterdrückt.

Tabelle 2

Kontrollwert = 100 %

III. Hemmung der Proliferation und Cytokin-Sekretion Mitogen-behandelter T-Zellen

Bei Mitogen-behandelten T-Zellen und bei einer Mischung (10:1) Mitogen-behandelter T-Zellen und Monocyten wurde ebenfalls die Proliferation der Zellen und die Sekretion der Cytokine unterdrückt. Ferner wurde bei IL-4-behandelten B-Zellen deren Proliferation und die Sekretion von Immunglobulinen unterdrückt.

IV. Beeinflussung der graft-versus-host disease

Bestrahlte Mäuse (BG-Stamm) wurden mit syngenetischen (BG) oder allogenetischen (Balb/c) Knochenmark- und Milzzellen rekonstituiert. Die nur bestrahlten Mäuse gingen alle während der ersten 10 Tage ein. Die mit syngenetischen Knochenmark- und Milzzellen rekonstituierten Tiere überlebten alle mehr als 60 Tage. Mäuse, die mit allogenetischen Zellen rekonstituiert wurden, entwickelten das graft-versus-host disease-Syndrom. Falls die Tiere mit der Verbindung A behandelt wurden (200 mg/kg täglich) wurde das Syndrom in seiner Entwicklung verzögert.

Das p-[2-(5,6,7,8-Tetrahydro-5,5,8,8,-tetramethyl-2-(naphthyl)propenyl]phenol kann für den erfindungsgemässen Zweck systemisch oder topisch, vorzugsweise systemisch, insbesondere enteral und speziell oral, verabreicht werden.

Die verabreichte Dosis richtet sich nach den Bedürfnissen des Patienten und wird vom behandelnden Arzt individuell bestimmt. Im allgemeinen kommt eine tägliche Dosis von etwa 1 mg bis etwa 20 mg, vorzugsweise von etwa 3 mg bis etwa 10 mg, pro kg Körpergewicht des Patienten in Betracht. Die Dosis kann als Einzeldosis oder auf mehrere Teildosen verteilt, verabreicht werden.

Als Verabreichungsform kommen die für die systemische Verabreichung üblichen festen oder flüssigen Darreichungsformen in Betracht, z.B. Suppositorien oder als feste orale Darreichungsformen Kapseln, Tabletten, Dragées, Pillen, Puder, Granulate und dergleichen, als flüssige orale Darreichungsformen Lösungen, Sirupe, Suspensionen, Elixire und dergleichen und als parenterale Darreichungsformen Infusions- oder Injektionslösungen, die intravenös oder intramuskulär injiziert werden können.

Typische Präparate für die systemische Verabreichung sind Kapseln und Tabletten, die etwa 50-500 mg Wirkstoff enthalten, oder Suspensionssyrup mit 50-200 mg Wirkstoff pro ml.

Zur topischen Verabreichung geeignet sind Lösungen, Lotions, Suspensionen, Salben, Crèmes, Gels, mikronisierte Puder, Aerosole und dergleichen. Diese Präparate enthalten zweckmässig 0,1-10 Gew.%, vorzugsweise 0,5-5 Gew.% Wirkstoff.

Die Herstellung der oben genannten systemischen und topischen Gebrauchsformen kann in üblicher Weise, z.B. anhand der nachstehenden Beispiele erfolgen.

Beispiel 1

Hartgelatinekapseln enthaltend die folgenden Bestandteile können wie folgt hergestellt werden:

| Bestandteile | mg/Kapsel |
|---|---|
| 1. sprühgetrocknetes Pulver enthaltend 75% Verbindung A | 100 |
| 2. Natriumdioctylsulfosuccinat | 0,1 |
| 3. Natriumcarboxymethylcellulose | 2,4 |
| 4. mikrokristalline Cellulose | 43,0 |
| 5. Talk | 4,0 |
| 6. Magnesiumstearat | 0,5 |
| Total | 150 |

Verfahren:

Das sprühgetrocknete Pulver, das auf dem Wirkstoff, Gelatine und mikrokristalliner Cellulose basiert und eine mittlere Korngrösse des Wirkstoffes von <1 $\mu$ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel 2

Tabletten, enthaltend die folgenden Bestandteile, können wie folgt hergestellt werden:

| Bestandteile | mg/Tablette |
|---|---|
| 1. Verbindung A als feingemahlenes Pulver | 250 |
| 2. Milchzucker pulv. | 50 |
| 3. Maisstärke weiss | 30 |
| 4. Povidone K30 | 4 |
| 5. Maisstärke weiss | 106 |
| 6. Talk | 8 |
| 7. Magnesiumstearat | 2 |
| Total | 450 |

Verfahren:

Der feingemahlene Wirkstoff wird mit Milchzucker pulv. und Maisstärke weiss gemischt. Die Mischung wird mit einer wässrigen Lösung von Povidone K30 befeuchtet und geknetet, und die resultierende Masse granuliert, getrocknet und gesiebt. Das Granulat wird mit Maisstärke weiss (2.Teil), Talk und Magnesiumstearat vermischt und zu Tabletten geeigneter Grösse verpresst.

Beispiel 3

Weichgelatinekapseln, enthaltend die folgenden Bestandteile, können wie folgt hergestellt werden:

| Bestandteile | mg/Kapsel |
|---|---|
| 1. Verbindung A | 250 |
| 2. Triglycerid | 700 |
| Total | 950 |

Verfahren:

100 g Verbindung A werden unter Rühren, Inertbegasung und Lichtschutz in 280 g mittelkettigem Triglycerid homogenisiert. Die Masse wird als Kapselfüllmasse zu Weichgelatinekapseln à 250 mg Wirkstoff verarbeitet.

Beispiel 4

Ein Suspensionssirup kann die folgende Zusammensetzung aufweisen:

| | |
|---|---|
| 1. Verbindung A | 800,0 mg |
| 2. Hydroxypropylmethylcellulose 2910/3 cp | 100,0 mg |
| 3. AVICEL RC 591 | 50,0 mg |
| 4. Antifoam C | 5,0 mg |
| 5. Sorbit Sirup 70% | 400,0 mg |
| 6. Propylenglycol | 250,0 mg |
| 7. Zucker krist. | 125,0 mg |
| 8. SYLOID 244 | 20,0 mg |
| 9. Artificial Orange Flavour | 40,0 mg |
| 10. Nipagin | 4,5 mg |
| 11. Nipasol | 0,5 mg |
| 12. Citronensäure q.s. ad pH 6,25 | ca. 1,225 mg |
| 13. Wasser entsalzt | ad 5,0 ml |

Beispiel 5

Eine Fettsalbe, enthaltend die folgenden Bestandteile, kann wie folgt hergestellt werden:

| Bestandteile | |
|---|---|
| 1. Verbindung A, feingemahlen | 3,0 g |
| 2. Paraffinöl, intern dickflüssig | 30,0 g |
| 3. Lunacera M | 15,0 g |
| 4. Ricinusöl, gehärtet | 5,0 g |
| 5. Vaseline, weiss | ad 100 g |

Verfahren:

Sämtliche Hilfsstoffe werden in der Wärme gemischt und auf Raumtemperatur kaltgerührt. Der Wirkstoff wird kalt unter Lichtschutz mit der auf diese Weise erhaltenen Mischung homogen angerieben.

Beispiel 6

Eine Fettcrème, enthaltend die folgenden Bestandteile, kann wie folgt hergestellt werden:

| Bestandteile | | |
|---|---|---|
| 1. Verbindung A, feingemahlen | 3,0 g | |
| 2. Vaseline, weiss | 30,0 g | |
| 3. Wachs, weiss | 5,0 g | Fettphase |
| 4. Paraffinöl, intern dickflüssig | 20,0 g | |
| 5. Dehymuls E | 9,0 g | |
| 6. Benzoesäure | 0,2 g | |
| 7. entmineralisiertes Wasser | ad 100,0 g | Wasserphase |

Verfahren:

Fett- und Wasserphase werden zu einer Fettcrème verarbeitet. Der Wirkstoff wird bei Raumtemperatur unter Lichtschutz mit dieser Fettcrème homogen angerieben.

Beispiel 7

Eine Vanishingcrème (Typ O/W-Emulsion), enthaltend die folgenden Bestandteile, kann wie folgt hergestellt werden:

| Bestandteile | | |
|---|---|---|
| 1. Verbindung A, feingemahlen | 3,0 g | |
| 2. Glycerinmonostearat | 17,0 g | |
| 3. Deltyl extra | 4,0 g | |
| 4. Tween 60 | 4,0 g | Fettphase |
| 5. Span 60 | 4,0 g | |
| 6. Silikonöl AR 20 | 2,0 g | |
| 7. Propylenglykol | 10,0 g | |
| 8. Benzoesäure, rein | 0,2 g | Wasserphase |
| 9. entmineralisiertes Wasser | ad 100,0 g | |

Verfahren:

Fett- und Wasserphase werden zu einer Crème verarbeitet. Der Wirkstoff wird bei Raumtemperatur unter Lichtschutz mit dieser Crème homogen angerieben.

Beispiel 8

Ein hydrophiles Gel, enthaltend die folgenden Bestandteile, kann wie folgt hergestellt werden:

| Bestandteile | |
|---|---|
| 1. Verbindung A, feingemahlen | 3,0 g |
| 2. Carbopol 940 | 2,5 g |
| 3. Propylenglykol | 50,0 g |
| 4. Aethanol, 94% | ad 100 g |

Verfahren:

Der Wirkstoff wird unter Lichtschutz in die Mischung Propylenglykol/Aethanol, 94%ig, eingearbeitet. Carbopol 940 wird bis zur vollständigen Gelierung eingerührt.

Beispiel 9

8

Eine Lotion, enthaltend die folgenden Bestandteile, kann wie folgt hergestellt werden:

| Bestandteile | |
|---|---|
| 1. Verbindung A, feingemahlen | 3,0 g |
| 2. Carbopol 934 | 0,6 g |
| 3. Natriumhydroxid | q.s. ad pH 6 |
| 4. Aethanol, 94% | 50,0 g |
| 5. entmineralisiertes Wasser | ad 100,0 g |

Verfahren:

Der Wirkstoff wird unter Lichtschutz in die Mischung Aethanol, 94%ig/Wasser eingearbeitet. Carbopol 934 wird bis zur vollständigen Gelierung eingerührt und der pH-Wert mit Natriumhydroxid eingestellt.

**Ansprüche**

1. Verwendung von p-[2-(5,6,7,8-Tetrahydro-5,5,8,8,-tetramethyl -2-(naphthyl)propenyl]phenol zur Herstellung eines Heilmittels für die Behandlung und Prophylaxe von Erkrankungen, die durch physiologische oder pathologische Veränderungen der Aktivitäten des Immunsystems hervorgerufen werden.

2. Verwendung des p-[(E)-2-(5,6,7,8-Tetrahydro -5,5,8,8-tetramethyl-2-naphthyl)propenyl]phenols gemäss Anspruch 1 oder 2.

3. Verfahren zur Herstellung eines Arzneimittels, wobei der Wirkstoff p-[2-(5,6,7,8-Tetrahydro-5,5,8,8-tetramethyl-2-naphthyl)propenyl]phenol, eventuell zusammen mit einem pharmazeutisch geeigneten Trägermaterial und sonstigen Hilfsstoffen, in eine für therapeutische Zwecke anwendbare Form gebracht wird, dadurch gekennzeichnet, dass man ein Heilmittel für die Behandlung oder Prophylaxe von Krankheiten, die durch physiologische oder pathologische Veränderungen der Aktivitäten des Immunsystems hervorgerufen werden, herstellt, bestehend aus der Kombination des Wirkstoffs oder aus einem den Wirkstoff enthaltendem Präparat zusammen mit der Information, welche in direktem Zusammenhang mit der Behandlung oder Prophylaxe der erwähnten Krankheiten steht.

4. Verfahren zur Herstellung von pharmazeutischen Präparaten, welche als Wirkstoff p-[2-(5,6,7,8-Tetrahydro-5,5,8,8-tetramethyl-2-naphthyl)propenyl]phenol sowie übliche Träger enthalten, dadurch gekennzeichnet, dass man den auf bekannte Art hergestellten Wirkstoff mit üblichen Trägern vermischt und in ein Arzneimittel für die Behandlung oder Prophylaxe von Krankheiten, die durch physiologische oder pathologische Veränderungen der Aktivitäten des Immunsystems hervorgerufen werden, überführt.

5. Handelspackung, enthaltend p-[2-(5,6,7,8-Tetrahydro-5,5,8,8-tetramethyl-2-naphthyl)propenyl]phenol als pharmazeutischen Wirkstoff, zusammen mit Instruktionen für dessen Verwendung zur Behandlung oder Prophylaxe von Krankheiten, die durch physiologische oder pathologische Veränderungen der Aktivitäten des Immunsystems hervorgerufen werden.